# DEMANDE DE BREVET EUROPEEN

(11) **EP 0 547 971 A1**
(43) Date de publication de la demande: **23.06.1993**
(21) Numéro de dépôt: 92403432.5
(22) Date de dépôt: 17.12.1992
(51) Int. Cl.: C07D 317/54, C07D 317/52

(54) **Procédé d'hydroxyméthylation de molécules aromatiques activées**

(30) Priorité: 18.12.1991 GB 9126876
(71) Demandeur: ROUSSEL UCLAF, F-93230 Romainville (FR)
(72) Inventeur: Penny, Stuart John, Maltby, Cleveland TS8 0BB (GB); Valentine, Roger Harry, Guisborough, Cleveland PS14 8LE (GB)
(74) Mandataire: Vieillefosse, Jean-Claude

(57) **Abrégé**

L'invention décrit un nouveau procédé amélioré d'hydroxyméthylation d'une molécule aromatique activée, qui consiste à utiliser du formaldéhyde et un acide carboxylique. Des extensions de ce procédé à l'halogénométhylation d'une molécule aromatique activée ou à l'introduction d'un groupe aldéhyde dans une molécule aromatique activée sont également décrites. En particulier, on y trouve l'utilisation des produits résultants pour préparer les composés de formule (1) 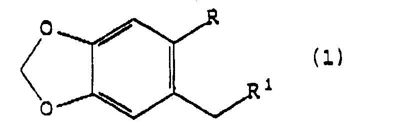 dans laquelle R représente un atome d'hydrogène ou un groupe alkyle en C₁-C₆ et R₁ représente un groupe polyalcoxyalkylène.

## Description

La présente invention se rapporte à l'hydroxyméthylation de composés aromatiques et à la transformation ultérieure des composés aromatiques hydroxyméthylés ainsi formés.

Dans un mode de réalisation particulièrement préféré, la présente invention se rapporte à un procédé amélioré pour préparer du butylate de pipéronyl de façon rentable en éliminant le risque de libération d'éther bis-chlorométhylique (BCME) toxique en tant que sous-produit nocif.

Dans un autre mode de réalisation, la présente invention se rapporte à un procédé de préparation de composés utiles dans les parfums ou les senteurs, par exemple le Cuménal, le pipéronal, l'anisaldéhyde, le t-butylbenzaldéhyde et le chlorométhylnaphtalène.

Dans un autre mode de réalisation, la présente invention se rapporte à un procédé de préparation de résines échangeuses d'ions par chlorométhylation. Les composés comme le butylate de pipéronyle sont représentatifs d'une classe de composés connus comme étant des agents synergiques insecticides représentés par la formule générale (1) : 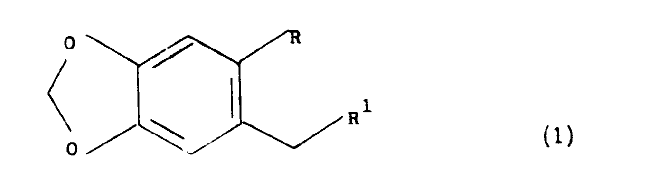
dans laquelle R est un hydrogène ou un groupe alkyle en Ci-Ce et R₁ est une chaîne latérale de type polyalcoxyalkylène. (Pour le butylate de pipéronyle, R = n-propyle et R₁ = nBuO(CH₂)₂O(CH₂)₂O-).

La préparation de ces composés a été décrite en détail dans la littérature (voir, par exemple, le brevet US n° 2 485 680 et le brevet britannique 632 589). Le procédé donnant ces composés (dont un exemple est le butylate de pipéronyle) est en général réalisé par réaction d'un halogénure tel que (2) avec le sel de sodium d'un polyalcoxyméthylènealcool (3).

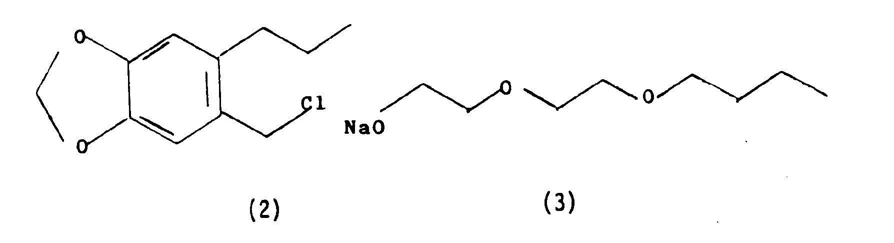

Les halogénures de formule (2) peuvent être facilement préparés par réaction de formaldéhyde et d'acide chlorhydrique sur du dihydrosafrole (4).

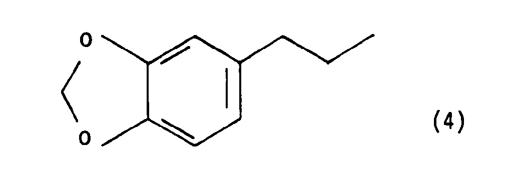

En variante, les composés tels que (1) peuvent être préparés par réaction de sels de sodium d'alcools ben- zyliques tels que (5) avec des halogénures de butylcarbityle tels que (6). (Brevet britannique 632 589).

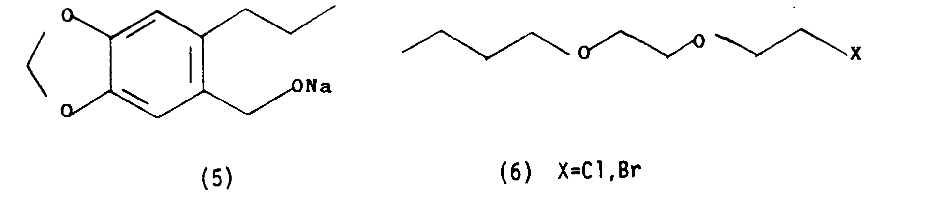

L'alcool benzylique nécessaire à (5) peut être préparé par transformation de l'halogénure (2) en son acétate, puis par saponification de l'ester.

Les deux voies ci-dessus font appel à une chlorométhylation du dihydrosafrole (4) avec du formaldéhyde et de l'acide chlorhydrique. On sait bien, dans la littérature, que l'éther bis-chlorométhylique (BCME) se forme lorsqu'on mélange du chlorure d'hydrogène (ou de l'acide chlorhydrique) et du formaldéhyde. (L.S. Frankel et al., Environmental Science & Technology, 1974, 8, (4), 356). Le BCME est un agent cancérigène respiratoire puissant (FJC Roe, The Lancet, 1985, 268; J. L. Gargus et al., Toxicology & Applied Pharmacology 1969, 15, 92; B. L. Van Duuren et al., J. Nat. Cancer. Inst., 1969, 43, 481; Chem. Eng. News, 1972, 50, (13) 55; Org. Reactions, 1972 (19),422, et Amer. Indust. Hygiene Assoc. J., 1972, 381). La limite d'exposition professionelle au BCME est actuellement de 0.001 ppm (0.005 mg m-³), qui correspond à l'une des limites les plus basses indiquées. Le fait d'éliminer la possibilité de former du BCME par un procédé de fabrication d'agents synergiques et de composés apparentés comme le butylate de pipéronyle présente un grand avantage par rapport aux voies connues actuellement.

Des améliorations des procédés existants ont été revendiquées (brevet US 2 878 265), mais la chimie de la chlorométhylation est encore au centre de la préparation des composés tels que (1). Ainsi, le brevet britannique 839 494 décrit une façon améliorée de fabriquer du chlorométhyldihydrosafrole et souligne un problème que l'on rencontre souvent dans la chimie de la chlorométhylation, à savoir la formation d'un diphénylméthane "dimère" (7).

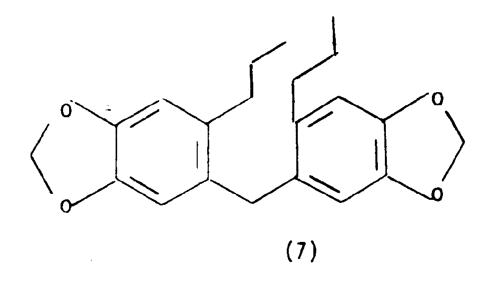

L'amélioration indiquée dans le brevet 839 494 consiste à éliminer pratiquement la formation de (7) et d'autres produits polymères. Cependent, le procédé emploie toujours du formaldéhyde et de l'acide chlorhydrique.

La réaction de Lederer Manasse permet de condenser un phénol ou éther aromatique avec du formaldéhyde pour donner un alcool benzylique (schéma 1).

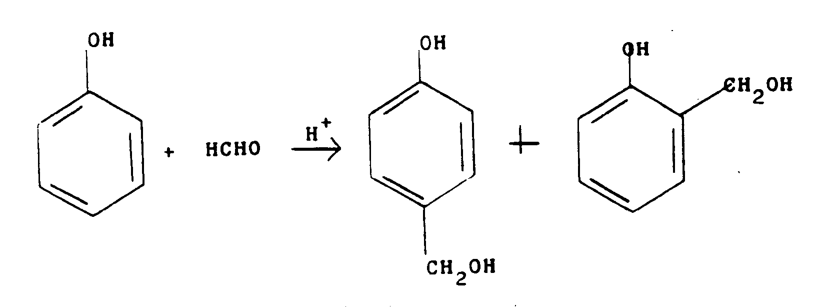

### Schéma 1

Les tentatives de mise en oeuvre de cette réaction sur du dihydrosafrole (4) se traduisent par la formation de rendements élevés en "dimère" (7) avec une gamme étendue de catalyseurs acides, notamment l'acide trifluoroacétique, l'acide phosphorique, l'acide formique, l'acide p-toluènesulfonique, l'acide sulfurique, à toutes sortes de concentrations.

On a maintenant découvert que l'on peut réaliser l'hydroxyméthylation de molécules aromatiques activées pour obtenir un dérivé protégé, le groupe protecteur étant ensuite éliminé pour donner le composé hydroxyméthylé, pratiquement sans aucune formation de sous-produit. Des composés tels que (1), par exemple le butylate de pipéronyle, peuvent être préparés par une voie qui évite la formation possible de BCME, qui minimise la formation de "dimère" (2) et qui permet donc de mettre en oeuvre un procédé industriel moins dangereux et plus attrayant.

Par conséquent, la présente invention fournit un procédé d'hydroxyméthylation d'une molécule aromatique activée, qui comprend la réaction de la molécule aromatique activée avec du formaldéhyde dans un acide carboxylique, en présence d'un catalyseur acide. L'acide carboxylique, est de préférence un acide carboxylique en C₂-C_{lO}, par exemple l'acide acétique, l'acide benzoïque ou l'acide naphtoïque. Le catalyseur acide ne contient de préférence pas d'atomes de chlore ou de brome, est avantageusement un acide minéral dilué, par exemple de l'acide sulfurique dilué, un acide sulfonique dilué, de l'acide trifluoroacétique, ou un acide de Lewis tel que le trifluorure de bore, de préférence de l'acide sulfurique dilué. L'acide carboxylique peut être sous la forme d'un dérivé acide protégé, comme un anhydride, par exemple l'anhydride acétique ou un anhydride mixte. La réaction est normalement réalisée à une température non extrême, par exemple comprise entre 20° et 100°C, avantageusement entre 20° et 90°C, et de préférence entre 60° et 90°C. La réaction peut être aussi réalisée dans des conditions hermétiques, par exemple dans un autoclave, auquel cas la réaction peut être réalisée à une température plus élevée, par exemple entre 100° et 150°C. Un solvant organique non miscible avec l'eau peut aussi être ajouté à la réaction pour donner un système à plusieurs phases; les alcanes ou cy- cloalcanes en C₅-Cₗₒ, par exemple l'hexane, constituent des solvants organiques non miscible qui conviennent pour être utilisés dans la réaction. Cette réaction donne la molécule aromatique hydroxyméthylée sous la forme d'un dérivé estérifié, le groupe hydroxyle libre peut être régénéré par élimination du groupe ester dans des conditions classiques, par exemple avec une solution aqueuse d'hydroxyde de sodium. Les groupes activants convenables pour la molécule aromatique englobent les groupes alkyle en Ci-Ce, alcoxy en Ci-Ce et polyalcoxy en Ci-Ce.

La molécule aromatique activée peut être une molécule hétérocyclique, polycarbocyclique ou polyhétéro- cyclique, ou un groupe phényle substitué par un groupe activant convenable. Les noyaux polyaromatiques contiennent avantageusement jusqu'à cinq, et de préférence deux ou trois, noyaux. Les molécules hétérocycliques contiennent avantageusement un maximum de cinq, et de préférence un à trois, hétéroatomes choisis parmi l'azote, l'oxygène et le soufre.

Par formaldéhyde, on entend l'utilisation de formaldéhyde aqueux, ou de préférence d'un polymère de formaldéhyde solide, comme le para-formaldéhyde, qui joue le rôle de précurseur de formaldéhyde. Le para- formaldéhyde est une forme particulièrement avantageuse à utiliser dans cette invention.

Ainsi, la réaction du dihydrosafrole (4) avec le formaldéhyde dans l'acide acétique, en présence de quantités catalytiques d'un acide, à une température non extrême, par exemple comprise entre 40°C et 100°C, de préférence de 60-80°C, donne des quantités appréciables d'acétate (8).

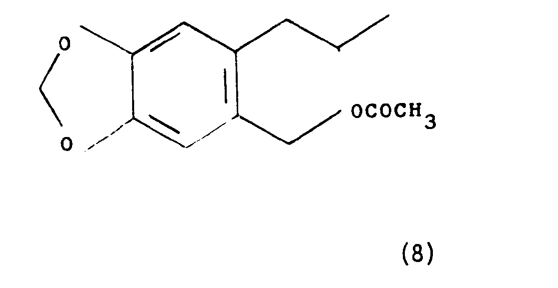

On a aussi découvert que l'on peut améliorer les rendements des intermédiaires (8) recherchés en réalisant la réaction dans un système à plusieurs phases. On fait ainsi réagir le dihydrosafrole (4) avec du formaldéhyde dans l'acide acétique, en présence d'une quantité catalytique d'acide sulfurique, en ajoutant un solvant non miscible non réactif, avantageusement un hydrocarbure tel que l'hexane, pour obtenir (8) avec un rendement proche de 60%.

Le solvant hydrocarbure non miscible de l'invention englobe n'importe quel hydrocarbure liquide de type alcane, cycloalcane, ou d'autres hydrocarbures saturés. Les solvants commodes englobent les pentanes, les kérosènes, et les distillats de pétrole aliphatiques analogues, ainsi que le cyclohexane. On utilise de préférence l'hexane.

Le composé hydrométhylé, ou son dérivé estérifié, peut être transformé en molécule halogénométhylée aromatique correspondante par des moyens classiques. Ainsi, sous le second de ses aspects, la présente invention procure un procédé d'halogénométhylation d'une molécule aromatique activée, ce procédé comprenant :
(i) l'hydroxyméthylation de la molécule aromatique activée par réaction de la molécule aromatique activée avec du formaldéhyde dans un acide carboxylique, en présence d'un catalyseuracide, puis éventuellement l'élimination du groupe acyle, et
(ii) l'halogénation du produit de (i) pour donner la molécule aromatique halogénométhylée correspondante. Avantageusement, l'halogénation est une chloration. Les agents d'halogénation convenables sont bien connus de l'homme de métier et englobent le chlorure de thionyle, l'oxychlorure de phosphore, le penta- chlorure de phosphore, le chlorure d'oxalyle. La réaction est avantageusement mise en oeuvre à une température non extrême, à savoir comprise entre -20 et 80°C, de préférence de 20-30°C.

Ainsi, on peut faire réagir l'acétate (8) avec le chlorure de thionyle, à une température d'environ 0°C à 50°C et, de préférence, à la température ambiante (20-30°C), pour obtenir le chlorométhyldihydrosafrole (2).

L'intermédiaire chlorométhylé (2) peut ensuite être transformé en butylate de pipéronyle à l'aide d'une chimie bien connue dans la littérature.

En variante, on peut oxyder le composé hydroxyméthylé ou son dérivé estérifié pour obtenir l'aldéhyde correspondant. Il s'agit là d'un procédé avantageux pour insérer une fonction aldéhyde sur un noyau polyaro- matique ou hétéroaromatique. Les exemples suivants sont fournis à titre d'illustration de l'invention.

### Exemple 1

### Préparation de l'acétate de 4, 5-méthylènedioxy-2-propyl-benzylique

On ajoute, en l'espace de 3 heures, un mélange de dihydrosafrole, 25 g, dans de l'acide acétique (25 g), tout en agitant, à un mélange d'acide acétique (230 g), de para-formaldéhyde (45 g) et d'acide sulfurique (10%, 1,6 g) maintenu à 70°C. Après que la réaction se soit révélée complète par chromatographie en phase vapeur, on élimine l'excès de paraformaldéhyde par filtration et on verse le filtrat dans de l'eau (450 ml). On ajoute ensuite une solution d'hydroxyde de sodium (20 ml d'une solution à 10%). Une extraction à l'éther, suivie d'une séchage sur du sulfate de magnésium, d'une filtration et de l'évaporation du solvant, donne une huile. On ajoute du méthanol (30 ml) et, après avoir refroidi à 0°C, on élimine par filtration un précipité incolore de "dimère" (7). L'évaporation du filtrat donne 12,7 g d'une huile que l'on purifie par distillation pour obtenir 7,3 g d'huile incolore qui est de l'acétate de 4,5-méthylène-dioxy-2-propylbenzyle (Eb 176-185°C sous 0,4 mm de Hg).

### Exemple 2

### Préparation du chlorure de 4,5-méthylènedioxy-2-propyl-benzyle (2) (chlorométhyldihydrosafrole)

On ajoute d'un seul coup du chlorure de thionyle (1,5 g) à une solution agitée d'acétate de 4,5-méthylè- nedioxy-2-propylbenzyle (8) (1,5 g) dans le dichlorométhane (20 ml). Après 2 heures d'agitation, la réaction semble être terminée par chromatographie en phase vapeur. Le mélange est ensuite versé dans de l'eau et la phase organique séparée et séchée sur du sulfate de magnésium. L'élimination du dichlorométhane après une filtration donne du chlorure de 4,5-méthylènedioxy-2-propylbenzyle (2) (1,1 g) sous forme d'une huile incolore. Analyse par chromatographie en phase vapeur : colonne garnie de SE30, à une température de 125°C programmée jusqu'a 200°C.

### Exemple 3

### Préparation du butylate de pipéronyle

On chauffe à 80°C pendant 2 heures un mélange réactionnel constitué de chlorure de 4,5-méthylènedioxy-2-propylbenzyle (2) (1,0 g) et du sel de potassium de butylcarbitol (2,0 g) dans de l'éther de pétrole (25 ml). On ajoute une aliquote supplémentaire (2,0 g) de sel de potassium de butylcarbitol et on poursuit le chauffage pendant encore une heure. On verse ensuite le mélange dans de l'eau. La séparation de la phase organique, suivie d'un séchage et d'une évaporation, donne du butylate de pipéronyle (2,8 g) sous forme d'une huile jaune limpide.

Analyse par chromatographie en phase vapeur : colonne garnie de SC30, on obtient un seul pic avec un temps de rétention identique à celui du butylate de pipéronyle authentique.

### Exemple 4

### Préparation de l'acétate de 4,5-méthylènedioxy-2-propylbenzyle (8)

On ajoute du dihydrosafrole (20 g), de l'acide acétique (100 g) et de l'eau (10 g) à un mélange d'acide acétique (250 ml), d'hexane (200 ml), d'anhydride acétique (40 g) et de para-formaldéhyde (40 g), auquel on a ajouté 1,7 g d'acide sulfurique à 40%. On maintient le mélange réactionnel à 60°C pendant 5 heures, puis on le refroidit à 20°C et on élimine l'excès de para-formaldéhyde par filtration. On ajoute ensuite de la saumure saturée (200 ml), puis de l'éther (100 ml). Le mélange est extrait à l'éther (3 fois) et les extraits combinés sont lavés avec de la saumure (3 fois). Après l'élimination du solvant sous pression réduite, on distille le produit sous vide, pour obtenir l'acétate de 4,5-méthylènedioxy-2-propylbenzyle (8) sous forme d'une huile incolore (Eb 154-165°C sous 2,0 mm de Hg). Rendement 59%.

## Revendications

1. Procédé d'hydroxyméthylation d'une molécule aromatique activée, qui comprend la réaction de la molécule aromatique activée avec du formaldéhyde dans un acide carboxylique, éventuellement sous la forme d'un dérivé protégé, en présence d'un catalyseur acide, pour donner la molécule aromatique hydroxyméthylée sous la forme d'un dérivé estérifié, éventuellement suivie de l'élimination du groupe ester par des méthodes classiques.

2. Procédé selon la revendication 1, dans lequel l'acide carboxylique est un acide carboxylique en C₂-Cₗₒ-3. Procédé selon la revendication 1 ou 2, dans lequel l'acide carboxylique est sous la forme d'un anhydride.

4. Procédé selon l'une quelconque des revendications 1 à 3, dans lequel un solvant organique non miscible dans l'eau est ajouté au mélange réactionnel pour donner un système à plusieurs phases.

5. Procédé d'halogénométhylation d'une molécule aromatique activée, qui comprend les étapes suivantes:
(i) hydroxyméthylation de la molécule aromatique activée par un procédé selon l'une quelconque des revendications 1 à 4, puis
(ii) halogénation par des méthodes classiques du produit aromatique hydroxyméthylé, ou de sa forme estérifiée, produit en (i).

6. Procédé pour introduire une fonction aldéhyde dans une molécule aromatique activée, qui comprend les étapes suivantes:
(i) hydroxyméthylation de la molécule aromatique activée par un procédé selon l'une quelconque des revendications 1 à 4, puis
(ii) oxydation par des méthodes classiques du produit aromatique hydroxyméthylé, ou de sa forme estérifiée, produit en (i).

7. Procédé selon l'une quelconque des revendications 1 à 6, dans lequel la molécule aromatique activée est un composé de formule (II) 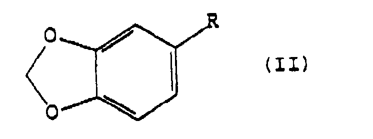
dans laquelle R représente un atome d'hydrogène ou un groupe alkyle en Ci-Ce.

8. Procédé selon la revendication 7, dans lequel la molécule aromatique activée est le composé de formule (4) 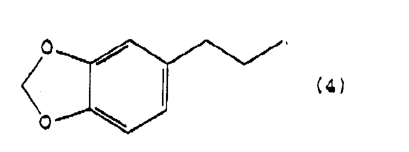
qui est le dihydrosafrole.

9. Procédé de préparation de composés de formule (1) 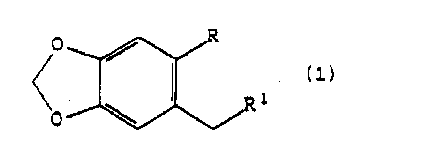
dans laquelle R est tel que défini dans la revendication 7 et R₁ représente un groupe polyalcoxyalkylène, qui comprend la réaction d'un composé de formule (III) 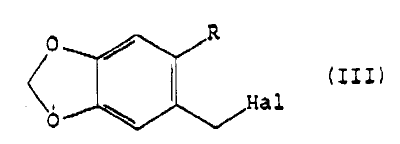
dans laquelle Hal représente un atome d'halogène, obtenu par un procédé selon la revendication 5, avec un composé de formule (IV) HO - R₁ (IV)
dans laquelle R₁ est tel que défini ci-dessus, éventuellement sous la forme de son sel de sodium.

10. Procédé selon la revendication 9, dans lequel on fait réagir un composé de formule (II), dans laquelle R représente un groupe n-propyle, avec un composé de formule (IV), dans laquelle R₁ représente un groupe de formule n-BuO(CH₂)₂O(CH₂)₂O-, pour donner le butylate de pipéronyle.
